Europäisches Patentamt

European Patent Office

Office européen des brevets

(11) Publication number: **0 072 229**
**A1**

## EUROPEAN PATENT APPLICATION

(21) Application number: **82304178.5**

(22) Date of filing: **09.08.82**

(51) Int. Cl.³: **A 62 B 18/02**
**// A61F9/06, A45D44/12**

(30) Priority: **07.08.81 GB 8124281**

(43) Date of publication of application: **16.02.83**
**Bulletin 83/7**

(84) Designated Contracting States: **BE DE FR GB LU NL SE**

(71) Applicant: **Siebe Gorman & Company Limited, 13 Park Street, Windsor Berkshire, SL4 1LU (GB)**

(72) Inventor: **Richards, John Bryan, 3 Edinburgh Close, Cwmbran Gwent Wales (GB)**
Inventor: **Pearce, Frederick Carlisle, 28 Westfield Avenue Malpas, Newport Gwent Wales (GB)**

(74) Representative: **Blanco White, Henry Nicholas et al, ABEL & IMRAY Northumberland House 303-306 High Holborn, London WC1V 7LH (GB)**

(54) **Improvements in and relating to face masks.**

(57) A face-mask comprises a single rigid transparent plastics moulding having an edge conforming closely to the shape of a human head, for example, the head of an average adult male caucasian, with a narrow flexible seal. A single face-mask can be made to fit up to 95% of people in the selected population.

The invention relates to face masks arranged in use to cover the entire face of the wearer with a single structure.

Such face masks have in the past been constructed largely of neoprene or other flexible material, arranged to form a seal with the face of the wearer all round the edges of the mask, with a transparent viewing plate and accessories such as air inlets and outlets set into the neoprene. Such a construction has always been considered necessary in the past to give the mask sufficient flexibility to accomodate faces of various shapes, although the difficulty of forming a satisfactory seal around the edge of the viewing plate has seriously restricted the design of such masks.

The invention provides a face mask comprising a single rigid unit arranged in use to cover the eyes, nose and mouth of a wearer and to conform to the shape of the wearer's face around the edges, and a flexible seal between the edge of the mask and the wearer's face.

The invention also provides a unit suitable for use as the said rigid unit in a face mask according to the invention.

Surprisingly, it has been found that it is possible to construct a single mask that will fit up to 99% of all male adults in a normal pupulation.

Preferably, the rigid unit is formed from a single moulding of rigid transparent plastics material, which is advantageously nylon or polycarbonate. Those parts of the mask through which the wearer does not need to be able to see may be painted or otherwise rendered opaque, or they may be left transparent.

Advantageously a spherically curved viewing portion is arranged close in front of the wearer's eyes, the lower part of the mask projecting further forward to allow room for an air supply demand valve or the like inside the rigid unit of the mask. Not only is the spherically curved viewing portion preferable to a flat or cylindrically curved viewing plate, it is also possible to reduce the volume of air inside the mask so that it is no longer necessary to provide a separate inner orinasal mask to reduce $CO_2$ content, and the breathing air supply can be arranged by means of a simple deflector to flow over the viewing portion to prevent misting up, avoiding the need for the more elaborate demisting arrangements that often had to be provided in the past.

The mask may be secured to the wearer's head with only four straps, two each side, eliminating the top centre strap that was usually necessary with flexible masks and was apt to interfere with the fitting of a helmet on the wearer's head.

One form of one-piece nylon moulding for a face mask according to the invention will now be described by way of example with reference to the accompanying drawings, in which:

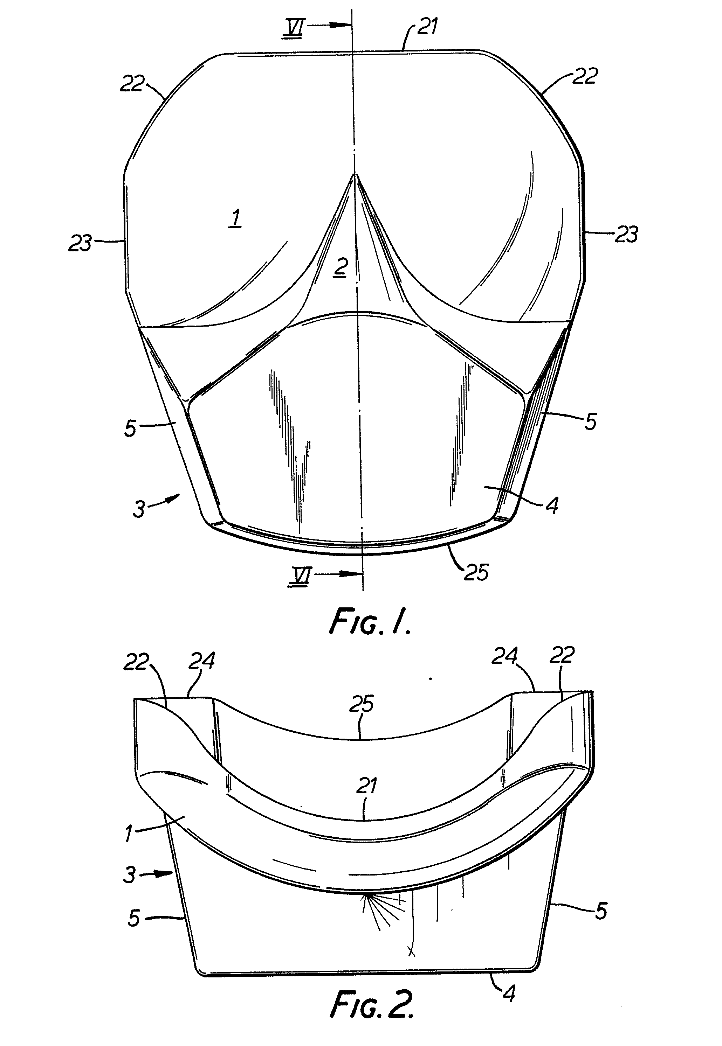

Fig. 1 is a front elevation view of the moulding;

Fig. 2 is a top plan view of the moulding;

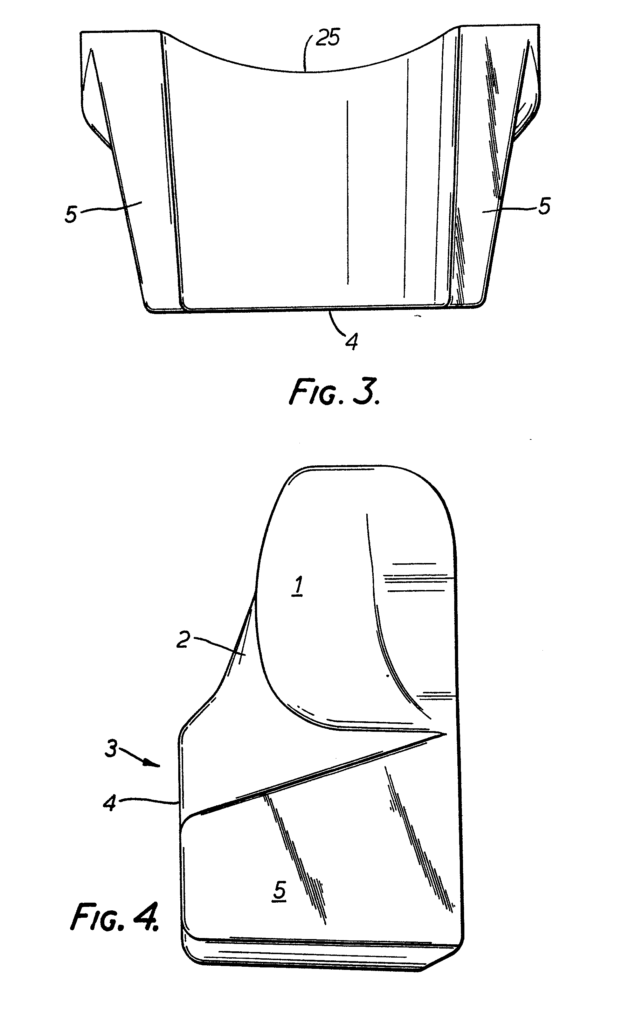

Fig. 3 is a bottom plan view of the moulding;

Fig. 4 is a side elevation view of the moulding;

Fig. 5 is a rear elevation view of the rim of the moulding; and

Fig. 6 is a side elevation view in section along the the line VI-VI of Fig. 1.

Referring to the drawings, the moulding comprises a spherically curved clear viewing portion 1 which is arranged in use to fit closely in front of the eyes of the wearer, with the centre of the notional sphere approximately centrally between the eyes, to give the wearer a good view. A raised conically curved portion 2 is provided to make room for the wearer's nose.

Below the viewing portion 2 is a projecting, faceted lower portion 3. In practice various apertures would usually be formed in the lower portion 3 to accommodate air inlet and outlet valves and other accessory equipment, and where appropriate an air supply demand valve, a microphone or the like can be arranged inside the lower portion, the rigid walls of which will give some protection against accidental damage. The flat facets of the lower portion 3 make the mounting of such accessory equipment comparatively easy. One preferred arrangement is to have an air supply demand valve mounted in the front wall 4 of the lower portion 3 and an outlet valve in one of the side walls 5, but the particular arrangement of the accessory equipment is not the subject of the present invention and only the basic moulding has been shown in the drawings.

A rim 6 extends round the entire periphery of the moulding. In use, a flexible seal (not shown), for example of rubber or neoprene, which may be of conventional design, is fitted along the rim 6. It will be appreciated that the seal will be comparatively narrow, because it need only span the gap between the edge of the mask and the face of the wearer, and the seal will be secured and supported by the rim 6. A light and simple structure for the seal can thus be achieved, although with the disadvantage that the mask will not fit a small minority of people with unusual facial shapes. A preferred form of seal is in the form of a neoprene tube inflated with air extending along the rim 6. The tube cross-section may have a circumference of about 100 mm and may be attached to the mask moulding by two flanges of the seal extending one on each side of the rim 6 and bonded to the material of the moulding by a suitable adhesive, advantageously a cyanoacrylate adhesive. Because an airtight bond can readily be achieved with adhesive, the joint with the mask moulding can also serve as a seam along the tube, greatly simplifying the construction. Instead, the seal may be held mechanically, for example, by a bead or flange on the seal being compressed into a channel formed in the rim 6.

In the complete mask four straps would be attached to the mask, on either side near the top and the bottom, to fasten the mask to the wearer's head. There is no need for the uncomfortable fifth strap over the top of the head that is usual with conventional masks. The straps may be attached to the mask by any conventional means.

It will be appreciated that the shape of the rim is of some importance, and the rim of the moulding illustrated is shaped as follows.  The shape is described with the moulding oriented with its plane of lateral symmetry vertical and the rear edge of the sides of the rim in a vertical plane, which will be referred to as the back plane, as it is shown in the drawings. Where ranges are specified, the values will normally vary in sympathy with one another. Where ranges are not specified, the dimensions may of course be varied at will.

A top facet 21 is a circular arc in a horizontal plane, with a radius of 76 to 78 mm, here 77 mm, and a centre 32 mm behind the back plane.  This leads at both sides into top corner facets 22, which have in front elevation an inside radius of 85 mm and are centred about an axis in the plane of symmetry 70 mm below the inside of the top facet 21.  In the other dimensions the top corner facets 22 provide a smooth transition from the curve of the top facet to straight, vertical side facets 23, which are 160 mm apart (but may be from 155 to 165 mm apart) between their insides and lie on the back plane 80 mm below the inside of the top facet 21, the side facets 23 meet cheek facets 24.  The cheek facets 24 converge downwards, each being angled at from 15 to 20°, here 17½°, to the vertical but still lying on the back plane.  The cheek facets 24 are joined at the bottom by a bottom facet 25.  The bottom facet 25 is curved in plan view with an inside radius of from 102 to 112 mm, here 110 mm, about an axis in the plane of symmetry 88 mm behind the back plane and in front elevation view with an inside radius of

0072229

135 mm about an axis in the plane of symmetry 40 mm below the inside of the top facet 21. The vertical height from the inside of the top facet 21 to the inside of the centre of the bottom facet is from 167 to 177 mm.

The viewing portion 1 is spherically curved with an inside radius of from 95 to 105 mm, here 100 mm, about a centre 32 mm behind the back plane and 50 mm below the inside of the top facet.

What we claim is:-

1. A face-mask comprising a single rigid unit arranged in use to cover the eyes, nose and mouth of a wearer and to conform to the shape of the wearer's face around the edge, and a flexible seal between the edge of the mask and the wearer's face.

2. A face-mask as claimed in claim 1, wherein the edge portion of the rigid unit extends generally rearwards, with respect to a standing wearer, throughout the circumference thereof.

3. A face-mask as claimed in claim 1 or claim 2, wherein the edge of the rigid unit is generally octagonal, having top, bottom, left and right side, and upper and lower left and right corner portions.

4. A face-mask as claimed in claim 3, wherein the two upper corner portions are curved and form a gradual transition from the top portion to the respective side portions.

5. A face-mask as claimed in claim 3 or claim 4, wherein the top portion is outwardly concave with a radius of from 76 to 78 mm, the bottom portion is outwardly concave with a radius of 105 to 115 mm, both as seen in plan view, the two lower corner portions converge downwards at angles of 15 to 20 degrees to the vertical, as seen in front elevation view, the vertical height between the centres of the top and bottom edge portions is from 167 to 177 mm, and the width between the side edge portions is from 155 to 165 mm, all views being taken with reference to a standing wearer, and wherein an upper portion of the unit comprises a spherically curved transparent portion having an external radius of from 97 to 107 mm arranged in use to be centred on a point between the eyes of the wearer.

6. A face-mask as claimed in any one of claims 1 to 5, wherein the said rigid unit consists of a single piece of transparent plastics material having apertures therein for inhaled and exhaled air.

FIG. 1.

FIG. 2.

FIG. 3.

FIG. 4.

0072229

21

22        22

23        23

6

24        24

25

*Fig. 5.*

22    6    21

23

1

2

24

4

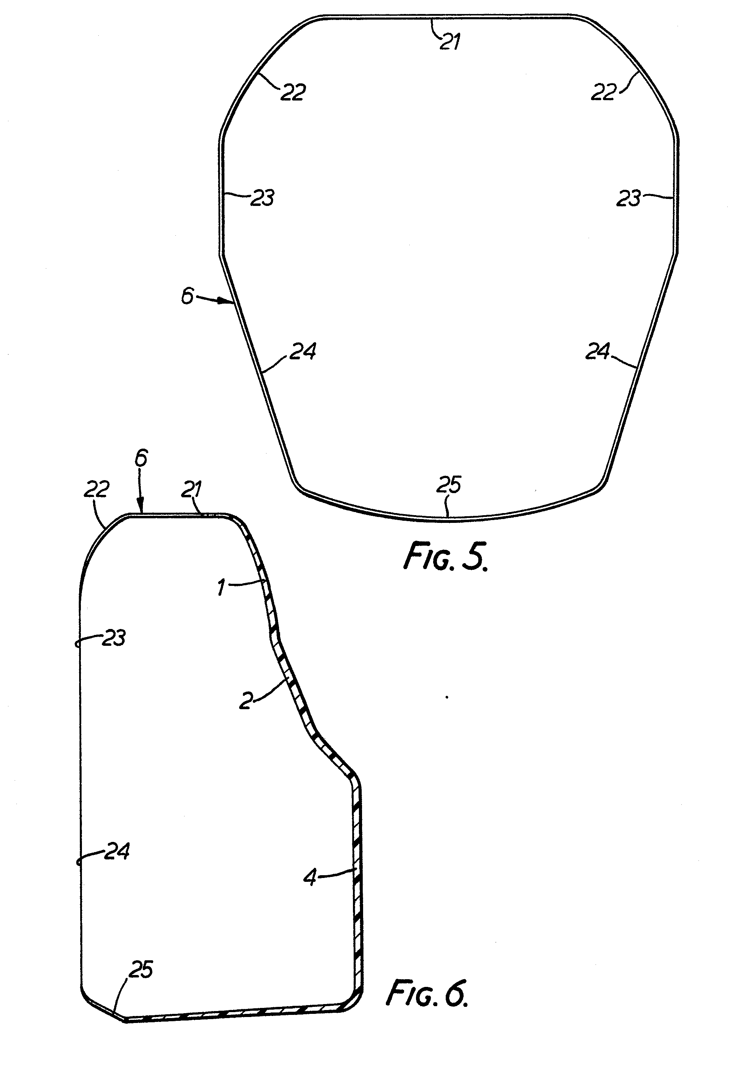

25    *Fig. 6.*

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | A 62 B 18/02 // |
| X | US-A-3 545 436 (AUTOMATIC SPRINKLER CORPORATION OF AMERICA) *Column 1, lines 4-20,64-75; column 2, entirely; column 3, lines 1-7; claims 1,5,6; figures* | 1,6 | A 61 F 9/06 A 45 D 44/12 |
| | --- | | |
| A | US-A-3 152 588 (F.J.ROGOWSKI) *Column 2, lines 1-31,68-72; column 3, lines 1-19; claim; figures* | 1-6 | |
| | --- | | |
| A | CH-A- 556 664 (R.MATHYS) *Column 1, lines 56-68; column 2, lines 1-5; figure 1* | 1,6 | |
| | --- | | |
| X,P | US-A-4 296 746 (SURGIKOS) *Column 2, lines 6-55; column 3, lines 31-68; column 4, lines 1,2; claims 1,2; figures 1-3* | 1,2,5, 6 | TECHNICAL FIELDS SEARCHED (Int. Cl. 3) |
| | --- | | A 41 G A 41 D |
| A | DE-C- 225 325 (WESTFALIA) *Page 1, lines 59-66; page 2, entirely; figures* | 1,2 | A 61 F A 62 B |
| | ----- | | |

The present search report has been drawn up for all claims

| Place of search THE HAGUE | Date of completion of the search 16-11-1982 | Examiner GARNIER F.M.A.C. |
|---|---|---|